# EUROPEAN PATENT SPECIFICATION

(11) **EP 4 577 513 B1**
(45) Date of publication and mention of the grant of the patent: **29.04.2026**
(21) Application number: 23757910.7
(22) Date of filing: 17.08.2023
(51) Int. Cl.: C07C 29/84, C07C 31/38

(54) **PROCESS FOR DISTILLATIVE RECOVERY OF 1,1,1,3,3,3-HEXAFLUORO-2-PROPANOL (HFIP) FROM MIXTURES WITH 4- SUBSTITUTED 1,2,3,4-TETRAHYDROQUINOLINES**
VERFAHREN ZUR DESTILLATIVEN RÜCKGEWINNUNG VON 1,1,1,3,3,3-HEXAFLUOR-2-PROPANOL (HFIP) AUS MISCHUNGEN MIT 4-SUBSTITUIERTEN 1,2,3,4-TETRAHYDROCHINOLINEN
PROCÉDÉ DE RÉCUPÉRATION PAR DISTILLATION DE 1,1,1,3,3,3-HEXAFLUORO-2-PROPANOL (HFIP) À PARTIR DE MÉLANGES CONTENANT DES 1,2,3,4-TETRAHYDROQUINOLINES SUBSTITUÉS EN POSITION 4

(30) Priority: 23.08.2022 EP 22191604
(43) Date of publication of application: 02.07.2025
(73) Proprietor: Bayer Aktiengesellschaft, 51373 Leverkusen (DE)
(72) Inventor: SCHOTES, Christoph, 51373 Leverkusen (DE)
(74) Representative: BIP Patents
(86) International application number: PCT/EP2023/072730
(87) International publication number: WO 2024/041976

(56) References cited:
- WO-A1-2019/185541
- US-A1- 2004 147 779

## Description

The invention relates to a process for distillative recovery of 1,1,1,3,3,3-hexafluoro-2-propanol (in the following also denominated hexafluoroisopropanol or HFIP) from a mixture comprising 1,1,1,3,3,3-hexafluoro-2-propanol and at least one 4-substituted 1,2,3,4-tetrahydroquinoline of formula (Ia) or (Ib), specified below.

4-Substituted 1,2,3,4-tetrahydroquinolines are versatile intermediates in the synthesis of N-indanyl heteroaryl carboxamide fungicides, including the recently launched pyrazole carboxamide fungicide inpyrfluxam (EP 0 654 464, WO 2015/141564, WO 2019/185541, WO 2021/058457, WO 2021/058458). They can be obtained by hydrogenation of the corresponding 4-substituted 1,2-dihydroquinolines.

WO 2015/141564 describes a process for preparing optically active 4-substituted 1,2,3,4-tetrahydroquinolines, which process comprises the hydrogenation of the corresponding 4-substituted 1,2-dihydroquinolines in presence of a transition metal catalyst having an optically active ligand. The asymmetric hydrogenation of the 4-substituted NH-dihydroquinolines proceeded with moderate conversion rates (up to 62.6%) and enantioselectivity (up to 71.3% ee), whereas N-acetyl-dihydroquinolines gave even poorer conversion (up to 14%) and enantioselectivity (up to 31% ee).

WO 2019/185541, WO 2021/058457 and WO 2021/058458 disclose enantioselective hydrogenation of the corresponding 4-substituted 1,2-dihydroquinolines in presence of a specific chiral iridium (P,N)-ligand catalyst which provides improved conversion rates and enantioselectivity. Conducting the enantioselective hydrogenation in the presence of a suitable solvent improves performance thereof. A particular suitable solvent is highly polar hexafluoroisopropanol. Albeit commercially available, it's comparatively high price would be preventive for application thereof in an industrial scale production process in the agrochemicals field, unless the solvent can be efficiently recycled.

Brenek et al. report in Brenek, S. J.; Caron, S.; Chisowa, E.; Delude, M. P.; Drexler, M. T.; Ewing, M. D.; Handfield, R. E.; Ide, N. D.; Nadkami, D. V.; Nelson, J. D.; Olivier, M.; Perfect, H. H.; Phillips, J. E.; Teixeira, J. J.; Weekly, R. M.; Zelina, J. P., Development of a Practical and Convergent Process for the Preparation of Sulopenem. Org. Process Res. Dev. 2012, 16 (8), 1348-1359, multi-step preparation of Sulopenem, a β-lactam antibiotic. One reaction step is selective oxidation of 2-thioalkyl penems with urea-hydrogen peroxide in hexafluoroisopropanol. It is stressed that using hexafluoroisopropanol requires maximizing concentration and/or recycling this material to lose as little of this solvent as possible (p. 1353, bottom of left column). Brenek et al. propose use of heptanes cosolvent that enabled the development of a suitable recycling process for the expensive hexafluoroisopropanol via distillation (p. 1353, right column, 1^{st} and 2^{nd} paragraphs).

Unfortunately, separating hexafluoroisopropanol from 4-substituted 1,2,3,4-tetrahydroquinolines turned out to be particularly difficult. It is believed that this is due to formation of hydrogen bonds between hexafluoroisopropanol and 4-substituted 1,2,3,4-tetrahydroquinolines. While distillation allows recycling of part of the hexafluoroisopropanol, the required high recycling rate is not achieved, even if heptanes are used as a cosolvent as proposed by Brenek et al.. Distillation at harsh conditions, i.e. very high temperatures, is also not suitable, since at such conditions 4-substituted 1,2,3,4-tetrahydroquinolines are not fully stable and undesired decomposition products are formed.

Hence, it is an object of the present invention to provide an efficient process for recycling 1,1,1,3,3,3-hexafluoro-2-propanol from its mixture with at least one 4-substituted 1,2,3,4-tetrahydroquinoline, which allows to recover as much 1,1,1,3,3,3-hexafluoro-2-propanol as possible, preferably to an extent that the remaining amount of 1,1,1,3,3,3-hexafluoro-2-propanol in said mixture is below 10 % by weight, preferably below 5 % by weight, more preferred below 1 % by weight, more preferred below 0.5 % by weight, most preferred below 0.1 % by weight.

The object described above is achieved by a process for distillative recovery of 1,1,1,3,3,3-hexafluoro-2-propanol from an initial mixture comprising 1,1,1,3,3,3-hexafluoro-2-propanol and a compound of the formula (**Ia**) or (**Ib**), 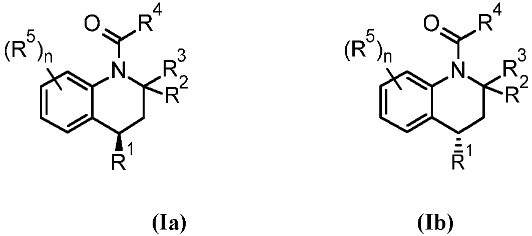 wherein
- R¹: is selected from the group consisting of C₁-C₆-alkyl, C₁-C₆-haloalkyl, C₁-C₆-alkoxy-C₁-C₆-alkyl, C₃-C₆-cycloalkyl, C₆-C₁₄-aryl, or C₆-C₁₄-aryl-C₁-C₄-alkyl,
wherein the C₁-C₆-alkyl, C₃-C₆-cycloalkyl and the C₁-C₆-alkoxy in the C₁-C₆-alkoxy-C₁-C₆-alkyl moiety, are optionally substituted by 1 to 3 substituents independently selected from the group consisting of halogen, C₁-C₄-alkoxy, C₁-C₄-haloalkyl, C₁-C₄-haloalkoxy and phenyl, wherein the phenyl may be substituted by one to five substituents selected independently from each other from halogen, C₁-C₄-alkyl, C₁-C₄-alkoxy, C₁-C₄-haloalkyl, and C₁-C₄-haloalkoxy, and
wherein the C₆-C₁₄-aryl and the C₆-C₁₄-aryl in the C₆-C₁₄-aryl-C₁-C₄-alkyl moiety in each case is unsubstituted or substituted by one to five substituents selected from the group consisting of halogen, C₁-C₄-alkyl, C₁-C₄-haloalkyl, C₁-C₄-alkoxy and C₁-C₄-haloalkoxy,
- R² and R³: are the same and are selected from the group consisting of hydrogen, C₁-C₆-alkyl, C₁-C₆-haloalkyl and C₁-C₆-alkoxy-C₁-C₆-alkyl,
or
- R² and R³: together with the carbon which they are bound to, form a C₃-C₆-cycloalkyl ring,
- R⁴: is hydrogen, C₁-C₆-alkyl, C₁-C₆-haloalkyl, C₁-C₆-alkoxy, C₁-C₆-haloalkoxy, C₁-C₆-alkylamino, C₂-C₆-alkenyl, C₂-C₆-alkynyl, C₃-C₆-cycloalkyl, C₃-C₆-cycloalkyl-C₁-C₄-alkyl, C₂-C₆-alkenyloxy, 9-flurorenylmethyleneoxy, C₆-C₁₄-aryl, C₆-C₁₄-aryloxy, C₆-C₁₄-aryl-C₁-C₄-alkyloxy or C₆-C₁₄-aryl-C₁-C₄-alkyl,
wherein the C₆-C₁₄-aryl as such or as part of a composite substituent is unsubstituted or substituted by one to five substituents selected from the group consisting of halogen, C₁-C₄-alkyl, C₁-C₄-haloalkyl, C₁-C₄-alkoxy and C₁-C₄-haloalkoxy,
- n: is 0, 1, 2, 3 or 4,

each substituent R⁵, if present, is independently selected from the group consisting of halogen, C₁-C₆-alkyl, C₁-C₆-haloalkyl, C₁-C₆-alkoxy, hydroxyl, amino and -C(=O)-C₁-C₆-alkyl,
characterized in that a protic solvent selected from the group consisting of water, alcohols having a boiling point at 1 bar of 115 °C or less, and mixtures thereof is added to the initial mixture to obtain a distillation mixture, and
the distillation mixture is subjected to distillation.

Surprisingly, addition of the protic solvent allows distillative recovery of 1,1,1,3,3,3-hexafluoro-2-propanol from its mixture with a compound of the formula (Ia) or (Ib) to such extend that the remaining amount of 1,1,1,3,3,3-hexafluoro-2-propanol in said mixture is below 10 % by weight, and even below 0.1 % by weight.

### Definitions

In the definitions of the symbols given in the above formulae, collective terms were used, which are generally representative of the following substituents:
Halogen: fluorine, chlorine, bromine or iodine, preferably fluorine, chlorine or bromine, and more preferably fluorine or chlorine.
Alkyl: saturated, straight-chain or branched hydrocarbyl substituents having 1 to 6, preferably 1 to 4 carbon atoms, for example (but not limited to) C₁-C₆-alkyl such as methyl, ethyl, propyl (n-propyl), 1-methylethyl (iso-propyl), butyl (n-butyl), 1-methylpropyl (sec-butyl), 2-methylpropyl (iso-butyl), 1,1-dimethylethyl (tert-butyl), pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Particularly, said group is a C₁-C₄-alkyl group, e.g. a methyl, ethyl, propyl, 1-methylethyl (isopropyl), butyl, 1-methylpropyl (sec-butyl), 2-methylpropyl (iso-butyl) or 1,1-dimethylethyl (tert-butyl) group. This definition also applies to alkyl as part of a composite substituent, for example C₃-C₆-cycloalkyl-C₁-C₄-alkyl, C₆-C₁₄-aryl-C₁-C₄-alkyl etc., unless defined elsewhere.
Alkenyl: unsaturated, straight-chain or branched hydrocarbyl substituents having 2 to 6, preferably 2 to 4 carbon atoms and one double bond in any position, for example (but not limited to) C₂-C₆-alkenyl such as vinyl, allyl, (E)-2-methylvinyl, (Z)-2-methylvinyl, isopropenyl, homoallyl, (E)-but-2-enyl, (Z)-but-2-enyl, (E)-but-1-enyl, (Z)-but-1-enyl, 2-methylprop-2-enyl, 1-methylprop-2-enyl, 2-methylprop-1-enyl, (E)-1-methylprop-1-enyl, (Z)-1-methylprop-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, 3- methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2- methylbut-1-enyl, (Z)-2-methylbut-1-enyl, (E)-1-methylbut-1-enyl, (Z)-1- methylbut-1-enyl, 1,1-dimethylprop-2-enyl, 1-ethylprop-1-enyl, 1-propylvinyl, 1- isopropylvinyl, (E)-3,3-dimethylprop-1-enyl, (Z)-3,3-dimethylprop-1-enyl, hex-5-enyl, (E)-hex-4- enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, 4-methylpent-4-enyl, 3-methylpent-4-enyl, 2-methylpent-4-enyl, 1- methylpent-4-enyl, 4-methylpent-3-enyl, (E)-3-methylpent-3-enyl, (Z)-3- methylpent-3-enyl, (E)-2-methylpent-3-enyl, (Z)-2-methylpent-3-enyl, (E)-1- methylpent-3-enyl, (Z)-1 -methylpent-3-enyl, (E)-4-methylpent-2-enyl, (Z)-4-methylpent-2-enyl, (E)-3-methylpent-2-enyl, (Z)-3-methylpent-2-enyl, (E)-2- methylpent-2-enyl, (Z)-2-methylpent-2-enyl, (E)-1 -methylpent-2-enyl, (Z)-1- methylpent-2-enyl, (E)-4-methylpent-1-enyl, (Z)-4-methylpent-1-enyl, (E)-3- methylpent-1-enyl, (Z)-3-methylpent-1 -enyl, (E)-2-methylpent-1 -enyl, (Z)-2-methylpent-1-enyl, (E)-1-methylpent-1-enyl, (Z)-1-methylpent-1-enyl, 3-ethylbut- 3-enyl, 2-ethylbut-3-enyl, 1-ethylbut-3-enyl, (E)-3-ethylbut-2-enyl, (Z)-3-ethylbut-2-enyl, (E)-2-ethylbut-2-enyl, (Z)-2-ethylbut-2-enyl, (E)-1-ethylbut-2-enyl, (Z)-1-ethylbut-2-enyl, (E)-3-ethylbut-1-enyl, (Z)-3-ethylbut-1-enyl, 2-ethylbut-1-enyl, (E)-1-ethylbut-1-enyl, (Z)-1-ethylbut-1-enyl, 2-propylprop-2-enyl, 1-propylprop-2- enyl, 2-isopropylprop-2-enyl, 1 -isopropylprop-2-enyl, (E)-2-propylprop-1-enyl, (Z)- 2-propylprop-1-enyl, (E)-1-propylprop-1-enyl, (Z)-1-propylprop-1-enyl, (E)-2- isopropylprop-1-enyl, (Z)-2-isopropylprop-1-enyl, (E)-1-isopropylprop-1-enyl, (Z)-1- isopropylprop-1-enyl, 1-(1,1-dimethylethyl)ethenyl, buta-1,3-dienyl, penta-1,4-dienyl, hexa-1,5-dienyl or methylhexadienyl. Particularly, said group is vinyl or allyl. This definition also applies to alkenyl as part of a composite substituent unless defined elsewhere.
Alkynyl: straight-chain or branched hydrocarbyl substituents having 2 to 6, preferably 2 to 4 carbon atoms and one triple bond in any position, for example (but not limited to) C₂-C₆-alkynyl, such as ethynyl, prop-1-ynyl, prop-2-ynyl, but-1-ynyl, but-2-ynyl, but-3-ynyl, 1-methylprop-2-ynyl, pent-1-ynyl, pent-2-ynyl, pent-3-ynyl, pent-4-ynyl, 2-methylbut-3-ynyl, 1 -methylbut-3-ynyl, 1-methylbut-2-ynyl, 3-methylbut-1-ynyl, 1-ethylprop-2-ynyl, hex-1-ynyl, hex-2-ynyl, hex-3-ynyl, hex-4-ynyl, hex-5-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl group. Particularly, said alkynyl group is ethynyl, prop-1-ynyl, or prop-2-ynyl. This definition also applies to alkynyl as part of a composite substituent unless defined elsewhere.
Alkylamino: monoalkylamino or dialkylamino, wherein monoalkylamino represents an amino radical having one alkyl residue with 1 to 6 carbon atoms attached to the nitrogen atom. Non-limiting examples include methylamino, ethylamino, n-propylamino, isopropylamino, n-butylamino and tert-butylamino. Wherein dialkylamino represents an amino radical having two independently selected alkyl residues with 1 to 6 carbon atoms each attached to the nitrogen atom. Non-limiting examples include N,N-dimethylamino, N,N-diethyl-amino, N,N-diisopropylamino, N-ethyl-N-methylamino, N-methyl-N-n-propylamino, N-isopropyl-N-n-propylamino and N-tert-butyl-N-methylamino.
Alkoxy: saturated, straight-chain or branched alkoxy substituents having 1 to 6, more preferably 1 to 4 carbon atoms, for example (but not limited to) C₁-C₆-alkoxy such as methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 2,2-dimethylpropoxy, 1-ethylpropoxy, 1,1-dimethylpropoxy, 1,2-dimethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy. This definition also applies to alkoxy as part of a composite substituent unless defined elsewhere.
Cycloalkyl: mono- or polycyclic, saturated hydrocarbyl substituents having 3 to 12, preferably 3 to 8 and more preferably 3 to 6 carbon ring members, for example (but not limited to) cyclopropyl, cyclopentyl, cyclohexyl and adamantyl. This definition also applies to cycloalkyl as part of a composite substituent, for example C₃-C₆-cycloalkyl-C₁-C₄-alkyl, unless defined elsewhere.
Haloalkyl: straight-chain or branched alkyl substituents having 1 to 6, preferably 1 to 4 carbon atoms (as specified above), where some or all of the hydrogen atoms in these groups are replaced by halogen atoms as specified above, for example (but not limited to) C₁-C₃-haloalkyl such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and 1,1,1-trifluoroprop-2-yl. This definition also applies to haloalkyl as part of a composite substituent unless defined elsewhere.
Haloalkenyl and haloalkynyl are defined analogously to haloalkyl except that, instead of alkyl groups, alkenyl and alkynyl groups are present as part of the substituent.
Haloalkoxy: straight-chain or branched alkoxy substituents having 1 to 6, preferably 1 to 4 carbon atoms (as specified above), where some or all of the hydrogen atoms in these groups are replaced by halogen atoms as specified above, for example (but not limited to) C₁-C₃-haloalkoxy such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy and 1,1,1-trifluoroprop-2-oxy. This definition also applies to haloalkoxy as part of a composite substituent, unless defined elsewhere.
Aryl: mono-, bi- or tricyclic aromatic or partially aromatic substituents having 6 to 14 carbon atoms, for example (but not limited to) phenyl, naphthyl, tetrahydronapthyl, indenyl and indanyl. The binding to the superordinate general structure can be carried out via any possible ring member of the aryl residue. Aryl is preferably selected from phenyl, 1-naphthyl, 2-naphthyl, 9-phenantryl und 9-antracenyl. Phenyl is particularly preferred.

The process according to the invention allows efficient separation of hexafluoroisopropanol from the compound of formula (**Ia**) or (**Ib**).

Preferably, compounds of the formula (**Ia**) or (**Ib**), in particular (**Ia**), are those, wherein the substituents are defined as follows:
- R¹: is C₁-C₆-alkyl or C₆-C₁₄-aryl-C₁-C₄-alkyl,
wherein C₆-C₁₄-aryl in the C₆-C₁₄-aryl-C₁-C₄-alkyl moiety is unsubstituted or substituted by one to five substituents selected from the group consisting of halogen, C₁-C₄-alkyl, C₁-C₄-haloalkyl, C₁-C₄-alkoxy and C₁-C₄-haloalkoxy,
- R² and R³: are the same and are selected from C₁-C₄-alkyl,
- R⁴: is C₁-C₄-alkyl, C₁-C₄-haloalkyl, C₁-C₄-alkoxy, C₁-C₄-haloalkoxy, phenyl or benzyl,
- n: is 0, 1 or 2,
each substituent R⁵, if present, is independently selected from the group consisting of halogen, C₁-C₆-alkyl and C₁-C₆-haloalkyl.

More preferred compounds of the formula (**Ia**) or (**Ib**), in particular (**Ia**), are those, wherein the substituents are defined as follows:
- R¹: is C₁-C₆-alkyl,
- R² and R³: are the same and are selected from C₁-C₄-alkyl,
- R⁴: is C₁-C₄-alkyl, C₁-C₄-haloalkyl, C₁-C₄-alkoxy, C₁-C₄-haloalkoxy, phenyl or benzyl,
- n: is 0, 1 or 2,
each substituent R⁵, if present, is independently selected from the group consisting of halogen, C₁-C₆-alkyl
and C₁-C₆-haloalkyl.

Even more preferred compounds of the formula (**Ia**) or (**Ib**), in particular (**Ia**), are those, wherein the substituents are defined as follows:
- R¹: is C₁-C₄-alkyl,
- R² and R³: are methyl,
- R⁴: is C₁-C₄-alkyl,
- n: is 0 or 1,
R⁵, if present, is fluorine.

Even more preferred compounds of the formula (**Ia**) or (**Ib**), in particular (**Ia**), are those, wherein the substituents are defined as follows:
- R¹: is methyl, ethyl or n-propyl,
- R² and R³: are methyl,
- R⁴: is C₁-C₄-alkyl,
- n: is 0 or 1,
R⁵, if present, is fluorine.

Even more preferred compounds of the formula (**Ia**) or (**Ib**), in particular (**Ia**), are those, wherein the substituents are defined as follows:
- R¹: is methyl or n-propyl,
- R² and R³: are methyl,
- R⁴: is methyl,
- n: is 0 or 1,
substituent R⁵, if present, is fluorine.

Most preferred compounds of the formula (**Ia**) or (**Ib**), in particular (**Ia**), are those, wherein the substituents are defined as follows:
- R¹: is methyl,
- R² and R³: are methyl,
- R⁴: is methyl,
- n: is 0.

The total amount of 1,1,1,3,3,3-hexafluoro-2-propanol and compound of formula **(Ia)** or **(Ib)** in the initial mixture subjected to the process according to the invention is preferably at least 90 % by weight based on the weight of the initial mixture, more preferred at least 95 % by weight, even more preferred at least 97 % by weight, even more preferred at least 98 % by weight, even more preferred at least 99 % by weight, most preferred at least 99.5 % by weight. In other words, the initial mixture comprises preferably less than 10 % by weight based on the weight of the initial mixture, more preferred less than 5 % by weight, even more preferred less than 3 % by weight, even more preferred less than 2 % by weight, even more preferred less than 1 % by weight, most preferred less than 0.5 % by weight of any component other than 1,1,1,3,3,3-hexafluoro-2-propanol and compound of formula **(Ia)** or **(Ib).** In case two, three or more compounds of formula **(Ia)** or **(Ib)** are present in the initial mixture, the ranges outlined above refer to total amount of 1,1,1,3,3,3-hexafluoro-2-propanol and all compounds of formula **(Ia)** or **(Ib).**

The amount of any protic solvent present in the initial mixture is preferably less than 0.5 % by weight based on the weight of the initial mixture, more preferred less than 0.2 % by weight, even more preferred less than 0.1 % by weight, even more preferred less than 0.05 % by weight, most preferred less than 0.01 % by weight.

Particularly preferred the initial mixture is the product mixture resulting from the synthesis of the compound of formula **(Ia)** or **(Ib)** via enantioselective hydrogenation of corresponding 4-substituted 1,2-dihydroquinolines in presence of a chiral iridium (P,N)-ligand catalyst as described in WO 2019/185541, WO 2021/058457 or WO 2021/058458.

The amount of 1,1,1,3,3,3-hexafluoro-2-propanol in the initial mixture is preferably at most 20 % by weight based on the weight of the initial mixture, more preferred at most 15 % by weight, even more preferred at most 12 % by weight, even more preferred at most 10 % by weight, even more preferred at most 5 % by weight.

Also initial mixtures comprising 1,1,1,3,3,3-hexafluoro-2-propanol in an amount of more than 20 % by weight based on the weight of the initial mixture can be subjected to the process according to the invention. However, if high amounts of 1,1,1,3,3,3-hexafluoro-2-propanol are present in the initial mixture, part thereof can be readily recovered by simple distillation without necessarily adding the protic solvent. Hence, if 1,1,1,3,3,3-hexafluoro-2-propanol is present in the initial mixture in an amount of more than 20 % by weight, it is preferred to remove part of the 1,1,1,3,3,3-hexafluoro-2-propanol by distillation without adding the protic solvent until the level of 1,1,1,3,3,3-hexafluoro-2-propanol reaches the threshold for efficient further distillation, e.g. until the amount of 1,1,1,3,3,3-hexafluoro-2-propanol is at most 20 % by weight. It is preferred to add the protic solvent at this stage and further distill the resulting distillation mixture.

The weight ratio of 1,1,1,3,3,3-hexafluoro-2-propanol to compound(s) of formula **(Ia)** or **(Ib)** in the initial mixture is preferably from 1 : 4 to 1 : 100, more preferred from 1: 6 to 1 : 100.

The protic solvent added to the initial mixture is preferably selected from the group consisting of water, methanol, ethanol, 1-propanol, 2-propanol, and mixtures thereof, more preferred from the group consisting of water, methanol, ethanol, 2-propanol, and mixtures thereof. Most preferred the protic solvent is water.

The amount of protic solvent added to the initial mixture is preferably from 1 to 300 % by weight based on the weight of the initial mixture, more preferred 1 to 200 % by weight, even more preferred 2 to 150 % by weight, even more preferred 2 to 100 % by weight, most preferred 3 to 50 % by weight. In case the protic solvent is a mixture of two, three or more solvents selected from the group consisting of water and alcohols having a boiling point at 1 bar of 115 °C or less, the ranges outlined above refer to the amount of the respective mixture, i.e. to the sum of amounts of the protic solvents being part of the protic solvent mixture.

Preferably, the distillation is performed at a pressure of from 0.05 to 1.2 bar, more preferred 0.3 to 1.1 bar, even more preferred 0.5 to 0.9 bar, most preferred 0.6 to 0.8 bar. Generally, reducing the pressure fosters distillation and allows to choose lower distillation temperatures.

The distillation is preferably conducted at a temperature of from 80 °C to 150 °C, more preferred 90 to 140 °C, most preferred 100 to 140 °C.

As readily understood by one skilled in the art distillation time can be widely varied depending on various factors, in particular scale of the distillation, i.e. amount of distillation mixture to be distilled, distillation pressure and distillation temperature, but is generally from 0.5 to 48 hours. The shorter the time needed to reach the required level of HFIP recovery the better for economic reasons. Hence, preferably the distillation time is less than 24 hours, more preferably less than 12 hours, most preferably less than 6 hours.

The process according to the invention may optionally be conducted in the presence of an aprotic additive selected from aliphatic hydrocarbons having 1 to 12 carbon atoms and mixtures thereof, which may be added to the initial mixture and/or the distillation mixture. Preferably, the aprotic additive is selected from C₁-C₁₀-alkanes, C₁-C₁₀-alkenes, C₃-C₁₂-cycloalkanes, C₃-C₁₂-cycloalkenes, and mixtures thereof, more preferably from pentane, hexane, heptane, octane, pentene, hexene, heptene, octene, cyclohexane, methylcyclohexane, dimethylcyclohexane, ethylcyclohexane, and mixtures thereof, even more preferably from heptane, cyclohexane, methylcyclohexane, and mixtures thereof. Most preferably, the aprotic additive is methylcyclohexane.

If present, the aprotic additive is preferably added in an amount to arrive at a content of aprotic additive in the distillation mixture from 1 to 400 % by weight based on the weight of the distillation mixture, preferably 1 to 300 % by weight, more preferred 5 to 200 % by weight, most preferred 5 to 100 % by weight. In case the aprotic additive is a mixture of two, three or more additives, the ranges outlined above refer to the amount of the respective mixture, i.e. to the sum of amounts of the aprotic additives being part of the aprotic additive mixture.

Preferably, the distillation is conducted using a distillation column.

In case an aprotic additive is added, it is preferred to use a water separator, preferably a Dean-Stark apparatus, downstream of the distillation column in order to separate an aprotic additive phase from the distillate and recirculate all or a part thereof to the distillation mixture.

### Abbreviations and Acronyms:

| n.d. | Not determined |
|---|---|
| h | Hour(s) |
| HFIP | Hexafluoroisopropanol |
| MCH | Methylcyclohexane |
| QNMR | Quantitative nuclear magnetic resonance spectroscopy |
| R-THQA | (4R)-1-(2,2,4-trimethyl-3,4-dihydroquinolin-1(2H)-yl)ethanone |
| w/w | By weight |

### Examples

### Examples 1-11 :

143 g hexafluoroisopropanol (HFIP) were mixed with 1357 g (4R)-1-(2,2,4-trimethyl-3,4-dihydroquinolin-1(2H)-yl)ethanone (R-THQA), already containing 0.4 % w/w of MCH and 0.5 % w/w HFIP, thus resulting in a final HFIP content of 10 % w/w. From this homogenous mixture, 200 g were taken for each experiment and the respective amount of additive(s) added thereto (Table 1). Each resulting distillation mixture was stirred at 110 or 130 °C oil bath temperature (respective temperature is indicated in Table 1) at 700 mbar for 1 h, using a 500 mL round-bottom flask and a standard distillation bridge with Liebig condenser. The distillate was collected. If a two-phasic distillate was received, the lower - HFIP containing - phase was separated. Weighing and analyzing the HFIP content in the distillate (or its lower phase respectively) by ¹⁹F quantitative NMR (internal standard: Alsystin) allows for the calculation of the percentage of recovered HFIP. The results are shown in Table 1.

**Table 1:**

| **Example** | **Temperature [°C]** | **Additive** | **Mass of HFIP containing distillate [g]** | **Content of HFIP in Distillate [% w/w] (QNMR)** | **% HFIP Recovered** |
|---|---|---|---|---|---|
| 1 | 110 | 20 g water | 18.9 | 27.3 | 26 |
| 2 | 110 | 10 g water + 10 g MCH | 8.4 | 34.1 | 14 |
| 3 | 110 | 20 g water + 20 g MCH | 20.1 | 23.3 | 23 |
| 4 | 130 | 20 g water | 21.5 | 27.7 | 30 |
| 5 | 130 | 10 g water + 10 g MCH | 11.2 | 41.7 | 23 |
| 6 | 130 | 20 g water + 20 g MCH | 21.8 | 25.7 | 28 |
| 7 | 110 | - | 0 | - | 0 |
| 8 | 110 | 20 g MCH | 0 | - | 0 |
| 9 | 110 | 20 g heptane | 0 | - | 0 |
| 10 | 130 | - | 0 | - | 0 |
| 11 | 130 | 20 g MCH | 0 | - | 0 |

Examples 1 to 11 show that addition of 10 g water (examples 2 and 5) and 20 g water (examples 1, 3, 4, 6), which corresponds to 5 and 10 % by weight, respectively, based on the weight of the initial HFIP / R-THQA mixture, allows distillative recovery of HFIP from the HFIP / R-THQA mixtures comprising 10 % by weight HFIP. Hence, HFIP content can be reduced to well below 10 % by weight even at comparatively mild distillation conditions (110 °C, 700 mbar) already after 1 h. In contrast, no HFIP is recovered under respective distillation conditions, if no solvent (examples 7 and 10) or the aprotic solvent MCH (examples 8 and 10) or heptane (example 9) is added.

### Examples 12-19:

From a mixture of 10 % w/w HFIP in R-THQA (similarly prepared as for examples 1-11), 200 g were taken for each experiment and the respective amount of additive added thereto (Table 2). The resulting mixture was stirred at 130 °C oil bath temperature at 700 mbar for 1 h, using a 500 mL round-bottom flask and a standard distillation bridge with Liebig condenser. The distillate was collected. Weighing and analyzing the HFIP content in the distillate by ¹⁹F quantitative NMR (internal standard: Alsystin) allows for the calculation of the percentage of recovered HFIP. The results are shown in Table 2.

**Table 2:**

| **Example** | **Temperature [°C]** | **Additive** | **Mass of HFIP containing distillate [g]** | **Content of HFIP in Distillate [%] (QNMR)** | **% HFIP Recovered** |
|---|---|---|---|---|---|
| 12 | 130 | 20 g methanol | 19.4 | 3.4 | 3.3 |
| 13 | 130 | 20 g ethanol | 15.7 | 4.7 | 3.7 |
| 14 | 130 | 20 g isopropanol | 11.7 | 4.5 | 2.6 |
| 15 | 130 | 20 g n-propanol | 4.9 | 6.3 | 1.5 |
| 16 | 130 | 20 g n-butanol | 0 | - | 0 |
| 17 | 130 | 20 g ethylenglycol | 0 | - | 0 |
| 18 | 130 | 20 g polyethylenglycol 600 | 0 | - | 0 |
| 19 | 130 | 20 g acetic acid | 0.1 | n.d. | ≤0.5 |

Examples 12 to 19 show that also addition of an alcohol having a boiling point at 1 bar of 115 °C or less (examples 12 to 15) allows distillative recovery of HFIP from the HFIP / R-THQA mixtures comprising 10 % by weight HFIP, while no or hardly any HFIP is recovered under respective distillation conditions, if n-butanol (boiling point at 1 bar of 118 °C, example 16) or another higher boiling alcohol (examples 17 and 18) or protic solvent (example 19) is added.

## Claims

1. A process for distillative recovery of 1,1,1,3,3,3-hexafluoro-2-propanol from an initial mixture comprising 1,1,1,3,3,3-hexafluoro-2-propanol and a compound of formula (Ia) or (Ib), 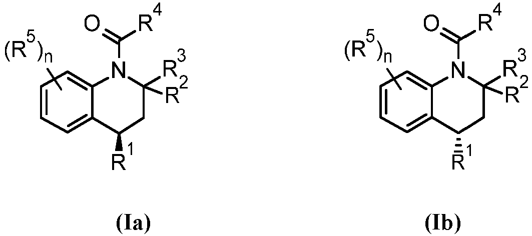 wherein
R¹ is selected from the group consisting of C₁-C₆-alkyl, C₁-C₆-haloalkyl, C₁-C₆-alkoxy-C₁-C₆-alkyl, C₃-C₆-cycloalkyl, C₆-C₁₄-aryl, or C₆-C₁₄-aryl-C₁-C₄-alkyl,
wherein the C₁-C₆-alkyl, C₃-C₆-cycloalkyl and the C₁-C₆-alkoxy in the C₁-C₆-alkoxy-C₁-C₆-alkyl moiety, are optionally substituted by 1 to 3 substituents independently selected from the group consisting of halogen, C₁-C₄-alkoxy, C₁-C₄-haloalkyl, C₁-C₄-haloalkoxy and phenyl, wherein the phenyl may be substituted by one to five substituents selected independently from each other from halogen, C₁-C₄-alkyl, C₁-C₄-alkoxy, C₁-C₄-haloalkyl, and C₁-C₄-haloalkoxy, and
wherein the C₆-C₁₄-aryl and the C₆-C₁₄-aryl in the C₆-C₁₄-aryl-C₁-C₄-alkyl moiety in each case is unsubstituted or substituted by one to five substituents selected from the group consisting of halogen, C₁-C₄-alkyl, C₁-C₄-haloalkyl, C₁-C₄-alkoxy and C₁-C₄-haloalkoxy,
R² and R³ are the same and are selected from the group consisting of hydrogen, C₁-C₆-alkyl, C₁-C₆-haloalkyl and C₁-C₆-alkoxy-C₁-C₆-alkyl,
or
R² and R³ together with the carbon which they are bound to, form a C₃-C₆-cycloalkyl ring,
R⁴ is hydrogen, C₁-C₆-alkyl, C₁-C₆-haloalkyl, C₁-C₆-alkoxy, C₁-C₆-haloalkoxy, C₁-C₆-alkylamino, C₂-C₆-alkenyl, C₂-C₆-alkynyl, C₃-C₆-cycloalkyl, C₃-C₆-cycloalkyl-C₁-C₄-alkyl, C₂-C₆-alkenyloxy, 9-flurorenylmethyleneoxy, C₆-C₁₄-aryl, C₆-C₁₄-aryloxy, C₆-C₁₄-aryl-C₁-C₄-alkyloxy or C₆-C₁₄-aryl-C₁-C₄-alkyl,
wherein the C₆-C₁₄-aryl as such or as part of a composite substituent is unsubstituted or substituted by one to five substituents selected from the group consisting of halogen, C₁-C₄-alkyl, C₁-C₄-haloalkyl, C₁-C₄-alkoxy and C₁-C₄-haloalkoxy,
n is 0, 1, 2, 3 or 4,
each substituent R⁵, if present, is independently selected from the group consisting of halogen, C₁-C₆-alkyl, C₁-C₆-haloalkyl, C₁-C₆-alkoxy, hydroxyl, amino and -C(=O)-C₁-C₆-alkyl,
wherein a protic solvent selected from the group consisting of water, alcohols having a boiling point at 1 bar of 115 °C or less, and mixtures thereof is added to the initial mixture to obtain a distillation mixture, and
the distillation mixture is subjected to distillation.

2. The process according to claim 1, wherein
R¹ is C₁-C₆-alkyl,
R² and R³ are the same and are selected from C₁-C₄-alkyl,
R⁴ is C₁-C₄-alkyl, C₁-C₄-haloalkyl, C₁-C₄-alkoxy, C₁-C₄-haloalkoxy, phenyl or benzyl,
n is 0, 1 or 2, and
each substituent R⁵, if present, is independently selected from the group consisting of halogen, C₁-C₆-alkyl and C₁-C₆-haloalkyl.

3. The process according to claim 1, wherein
R¹ is C₁-C₄-alkyl,
R² and R³ are methyl,
R⁴ is C₁-C₄-alkyl,
n is 0 or 1, and
R⁵ if present, is fluorine.

4. The process according to claim 1, wherein
R¹ is methyl,
R² and R³ are methyl,
R⁴ is methyl, and
n is 0.

5. The process according to any one of claims 1 to 4, wherein the total amount of 1,1,1,3,3,3-hexafluoro-2-propanol and compound of formula (Ia) or (Ib) in the initial mixture is at least 90 % by weight based on the weight of the initial mixture.

6. The process according to any one of claims 1 to 5, wherein the amount of 1,1,1,3,3,3-hexafluoro-2-propanol in the initial mixture is at most 20 % by weight based on the weight of the initial mixture.

7. The process according to any one of claims 1 to 6, wherein the protic solvent is selected from the group consisting of water, methanol, ethanol, 1-propanol, 2-propanol, and mixtures thereof.

8. The process according to any one of claims 1 to 7, wherein the protic solvent is water.

9. The process according to any one of claims 1 to 8, wherein the amount of protic solvent added to the initial mixture is from 1 % to 300 % by weight based on the weight of the initial mixture.

10. The process according to any one of claims 1 to 9, wherein the distillation is conducted at a pressure of from 0.05 bar to 1.2 bar.

11. The process according to any one of claims 1 to 10, wherein the distillation is conducted at a temperature of from 80 °C to 150 °C.

12. The process according to any one of claims 1 to 11, wherein the distillation mixture is distilled for 0.5 hours to 48 hours.

13. The process according to any one of claims 1 to 12, wherein an aprotic additive selected from aliphatic hydrocarbons having 1 to 12 carbon atoms, and mixtures thereof is added to the initial mixture or the distillation mixture.

14. The process according to claim 13, wherein the aprotic additive is added in an amount to arrive at a content of aprotic additive in the distillation mixture from 1 to 400 % by weight based on the weight of the distillation mixture.

15. The process according to any one of claims 1 to 14, wherein the distillation is conducted using a distillation column.

## Patentansprüche

1. Ein Verfahren zur destillativen Rückgewinnung von 1,1,1,3,3,3-Hexafluor-2-propanol aus einer Ausgangsmischung umfassend 1,1,1,3,3,3-Hexafluor-2-propanol und eine Verbindung der Formel (Ia) oder (Ib), 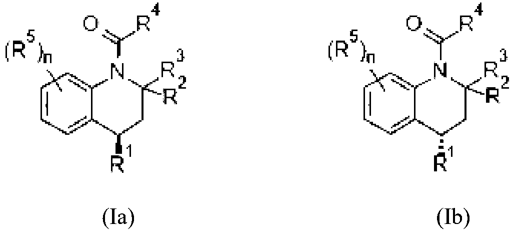 wobei
R¹ aus der Gruppe bestehend aus C₁-C₆-Alkyl, C₁-C₆-Halogenalkyl, C₁-C₆-Alkoxy-C₁-C₆-Alkyl, C₃-C₆-Cycloalkyl, C₆-C₁₄-Aryl oder C₆-C₁₄-Aryl-C₁-C₄-Alkyl ausgewählt ist,
wobei das C₁-C₆-Alkyl, das C₃-C₆-Cycloalkyl und das C₁-C₆-Alkoxy im C₁-C₆-Alkoxy-C₁-C₆-Alkyl-Rest optional durch 1 bis 3 Substituenten substituiert sind, die unabhängig voneinander aus der Gruppe bestehend aus Halogen, C₁-C₄-Alkoxy, C₁-C₄-Halogenalkyl, C₁-C₄-Halogenalkoxy und Phenyl ausgewählt sind, wobei das Phenyl durch ein bis fünf Substituenten substituiert sein kann, die unabhängig voneinander aus Halogen, C₁-C₄-Alkyl, C₁-C₄-Alkoxy, C₁-C₄-Halogenalkyl und C₁-C₄-Halogenalkoxy ausgewählt sind, und
wobei das C₆-C₁₄-Aryl und das C₆-C₁₄-Aryl im C₆-C₁₄-Aryl-C₁-C₄-Alkyl-Rest jeweils unsubstituiert oder durch ein bis fünf Substituenten substituiert sind, die aus der Gruppe bestehend aus Halogen, C₁-C₄-Alkyl, C₁-C₄-Halogenalkyl, C₁-C₄-Alkoxy und C₁-C₄-Halogenalkoxy ausgewählt sind,
R² and R³ gleich sind und aus der Gruppe bestehend aus Wasserstoff, C₁-C₆-Alkyl, C₁-C₆-Halogenalkyl und C₁-C₆-Alkoxy-C₁-C₆-Alkyl ausgewählt sind,
oder
R² and R³ zusammen mit dem Kohlenstoff, an den sie gebunden sind, einen C₃-C₆-Cycloalkylring bilden,
R⁴ Wasserstoff, C₁-C₆-Alkyl, C₁-C₆-Halogenalkyl, C₁-C₆-Alkoxy, C₁-C₆-Halogenalkoxy, C₁-C₆-Alkylamino, C₂-C₆-Alkenyl, C₂-C₆-Alkinyl, C₃-C₆-Cycloalkyl, C₃-C₆-Cycloalkyl-C₁-C₄-Alkyl, C₂-C₆-Alkenyloxy, 9-Fluorenylmethylenoxy, C₆-C₁₄-Aryl, C₆-C₁₄-Aryloxy, C₆-C₁₄-Aryl-C₁-C₄-Alkyloxy oder C₆-C₁₄-Aryl-C₁-C₄-Alkyl ist,
wobei das C₆-C₁₄-Aryl als solches oder als Teil eines zusammengesetzten Substituenten unsubstituiert oder durch ein bis fünf Substituenten substituiert ist, die aus der Gruppe bestehend aus Halogen, C₁-C₄-Alkyl, C₁-C₄-Halogenalkyl, C₁-C₄-Alkoxy und C₁-C₄-Halogenalkoxy ausgewählt sind,
n 0, 1, 2, 3 oder 4 ist,
jeder Substituent R⁵, falls vorhanden, unabhängig voneinander aus der Gruppe bestehend aus Halogen, C₁-C₆-Alkyl, C₁-C₆-Halogenalkyl, C₁-C₆-Alkoxy, Hydroxyl, Amin und -C(=O)-C₁-C₆-Alkyl ausgewählt ist,
wobei ein protisches Lösungsmittel aus der Gruppe bestehend aus Wasser, Alkoholen mit einem Siedepunkt bei 1 bar von 115 °C oder weniger und Mischungen davon der Ausgangsmischung zugesetzt wird, um eine Destillationsmischung zu erhalten, und
die Destillationsmischung einer Destillation unterzogen wird.

2. Das Verfahren nach Anspruch 1, wobei
R¹ C₁-C₆-Alkyl ist,
R² and R³ gleich sind und aus C₁-C₄-Alkyl ausgewählt sind,
R⁴ C₁-C₄-Alkyl, C₁-C₄-Halogenalkyl, C₁-C₄-Alkoxy, C₁-C₄-Halogenalkoxy, Phenyl oder Benzyl ist,
n 0, 1 oder 2 ist, und
jeder Substituent R⁵, falls vorhanden, unabhängig aus der Gruppe bestehend aus Halogen, C₁-C₆-Alkyl und C₁-C₆-Halogenalkyl ausgewählt ist.

3. Das Verfahren nach Anspruch 1, wobei
R¹ C₁-C₄-Alkyl ist,
R² and R³ Methyl sind,
R⁴ C₁-C₄-Alkyl ist,
n 0 oder 1 ist, und
R⁵ falls vorhanden, Fluor ist.

4. Das Verfahren nach Anspruch 1, wobei
R¹ Methyl ist,
R² and R³ Methyl sind,
R⁴ Methyl ist, und
n 0 ist.

5. Das Verfahren nach einem der Ansprüche 1 bis 4, wobei die Gesamtmenge an 1,1,1,3,3,3-Hexafluor-2-propanol und Verbindung der Formel (Ia) oder (Ib) in der Ausgangsmischung mindestens 90 Gew.-% bezogen auf das Gewicht der Ausgangsmischung beträgt.

6. Das Verfahren nach einem der Ansprüche 1 bis 5, wobei die Menge an 1,1,1,3,3,3-Hexafluor-2-propanol in der Ausgangsmischung höchstens 20 Gew.-% bezogen auf das Gewicht der Ausgangsmischung beträgt.

7. Das Verfahren nach einem der Ansprüche 1 bis 6, wobei das protische Lösungsmittel aus der Gruppe bestehend aus Wasser, Methanol, Ethanol, 1-Propanol, 2-Propanol und Mischungen davon ausgewählt ist.

8. Das Verfahren nach einem der Ansprüche 1 bis 7, wobei das protische Lösungsmittel Wasser ist.

9. Das Verfahren nach einem der Ansprüche 1 bis 8, wobei die Menge des protischen Lösungsmittels, die der Ausgangsmischung zugesetzt wird, 1 % bis 300 % Gew.-% bezogen auf das Gewicht der Ausgangsmischung beträgt.

10. Das Verfahren nach einem der Ansprüche 1 bis 9, wobei die Destillation bei einem Druck von 0,05 bar bis 1,2 bar durchgeführt wird.

11. Das Verfahren nach einem der Ansprüche 1 bis 10, wobei die Destillation bei einer Temperatur von 80 °C bis 150 °C durchgeführt wird.

12. Das Verfahren nach einem der Ansprüche 1 bis 11, wobei die Destillationsmischung für 0,5 Stunden bis 48 Stunden destilliert wird.

13. Das Verfahren nach einem der Ansprüche 1 bis 12, wobei ein aprotischer Zusatz aus aliphatischen Kohlenwasserstoffen mit 1 bis 12 Kohlenstoffatomen und Mischungen davon der Ausgangsmischung oder der Destillationsmischung zugesetzt wird.

14. Das Verfahren nach Anspruch 13, wobei der aprotische Zusatz in einer Menge zugesetzt wird, um einen Gehalt an aprotischem Zusatz in der Destillationsmischung von 1 bis 400 Gew.-% bezogen auf das Gewicht der Destillationsmischung zu erreichen.

15. Das Verfahren nach einem der Ansprüche 1 bis 14, wobei die Destillation unter Verwendung einer Destillationskolonne durchgeführt wird.

## Revendications

1. Un procédé pour 1a récupération distillative de 1,1,1,3,3,3-hexafluoro-2-propanol à partir d'un mélange initial comprenant 1,1,1,3,3,3-hexafluoro-2-propanol et un composé de formule (Ia) ou (Ib), 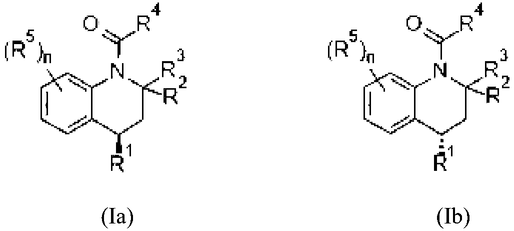 dans lequel
R¹ est sélectionnée parmi le group constitant en C₁-C₆-alkyle, C₁-C₆-haloalkyl, C₁-C₆-alkoxy-C₁-C₆-alkyle, C₃-C₆-cycloalkyle, C₆-C₁₄-aryl, ou C₆-C₁₄-aryl-C₁-C₄-alkyle,
dans lequel le C₁-C₆-alkyle, C₃-C₆-cycloalkyle et le C₁-C₆-alkoxy dans le C₁-C₆-alkoxy-C₁-C₆-alkyle, sont éventuellement substitués par 1 à 3 substituants sélectionnés indépendamment parmi le group constitant en halogène, C₁-C₄-alkoxy, C₁-C₄-haloalkyl, C₁-C₄-haloalkoxy et phényle, où le phényle peut être substitué par un à cinq substituants sélectionnés indépendamment les uns des autres parmi halogène, C₁-C₄-alkyle, C₁-C₄-alkoxy, C₁-C₄-haloalkyl, et C₁-C₄-haloalkoxy, et
dans lequel le C₆-C₁₄-aryl et le C₆-C₁₄-aryl dans le C₆-C₁₄-aryl-C₁-C₄-alkyle, dans chaque cas, est non substitué ou substitué par un à cinq substituants sélectionnés parmi le group constitant en halogène, C₁-C₄-alkyle, C₁-C₄-haloalkyl, C₁-C₄-alkoxy et C₁-C₄-haloalkoxy,
R² et R³ sont les mêmes et sont sélectionnés parmi le group constitant en hydrogène, C₁-C₆-alkyle, C₁-C₆-haloalkyl et C₁-C₆-alkoxy-C₁-C₆-alkyle,
ou
R² et R³ avec le carbone auquel ils sont liés, forment un cycle C₃-C₆-cycloalkyle,
R⁴ est hydrogène, C₁-C₆-alkyle, C₁-C₆-haloalkyl, C₁-C₆-alkoxy, C₁-C₆-haloalkoxy, C₁-C₆-alkylamino, C₂-C₆-alkényl, C₂-C₆-alkynyl, C₃-C₆-cycloalkyle, C₃-C₆-cycloalkyle-C₁-C₄-alkyle, C₂-C₆-alkényloxy, 9-flurorenylméthylèneoxy, C₆-C₁₄-aryl, C₆-C₁₄-aryloxy, C₆-C₁₄-aryl-C₁-C₄-alkyloxy ou C₆-C₁₄-aryl-C₁-C₄-alkyle,
dans lequel le C₆-C₁₄-aryl en tant que tel ou en tant que partie d'un substituant composite, est non substitué ou substitué par un à cinq substituants sélectionnés parmi le group constitant en halogène, C₁-C₄-alkyle, C₁-C₄-haloalkyl, C₁-C₄-alkoxy et C₁-C₄-haloalkoxy,
n est 0, 1, 2, 3 ou 4,
chaque substituant R⁵, si présent, est sélectionné indépendamment parmi le group constitant en halogène, C₁-C₆-alkyle, C₁-C₆-haloalkyl, C₁-C₆-alkoxy, hydroxyle, amino et -C(=O)-C₁-C₆-alkyle,
dans lequel un solvant protique sélectionné parmi le group constitant en eau, alcools ayant un point d'ébullition à 1 bar de 115 °C ou moins, et leurs mélanges, est ajouté au mélange initial pour obtenir un mélange de distillation, et
le mélange de distillation est soumis à la distillation.

2. Le procédé selon la revendication 1, dans lequel
R¹ est C₁-C₆-alkyle,
R² et R³ sont les mêmes et sont sélectionnés parmi C₁-C₄-alkyle,
R⁴ est C₁-C₄-alkyle, C₁-C₄-haloalkyle, C₁-C₄-alkoxy, C₁-C₄-haloalkoxy, phényle ou benzyle,
n est 0, 1 ou 2, et
chaque substituant R⁵, si présent, est sélectionné indépendamment parmi le groupe consistant en halogène, C₁-C₆-alkyle et C₁-C₆-haloalkyle.

3. Le procédé selon la revendication 1, dans lequel
R¹ est C₁-C₄-alkyle,
R² et R³ sont méthyle,
R⁴ est C₁-C₄-alkyle,
n est 0 ou 1, et
R⁵ si présent, est fluor.

4. Le procédé selon la revendication 1, dans lequel
R¹ est méthyle,
R² et R³ sont méthyle,
R⁴ est méthyle, et
n est 0.

5. Le procédé selon l'une quelconque des revendications 1 à 4, dans lequel la quantité totale de 1,1,1,3,3,3-hexafluoro-2-propanol et du composé de formule **(Ia)** ou **(Ib)** dans le mélange initial est d'au moins 90 % en poids par rapport au poids du mélange initial.

6. Le procédé selon l'une quelconque des revendications 1 à 5, dans lequel la quantité de 1,1,1,3,3,3-hexafluoro-2-propanol dans le mélange initial est d'au plus 20 % en poids par rapport au poids du mélange initial.

7. Le procédé selon l'une quelconque des revendications 1 à 6, dans lequel le solvant protique est sélectionné parmi le groupe consistant en eau, méthanol, éthanol, 1-propanol, 2-propanol, et leurs mélanges.

8. Le procédé selon l'une quelconque des revendications 1 à 7, dans lequel le solvant protique est l'eau.

9. Le procédé selon l'une quelconque des revendications 1 à 8, dans lequel la quantité de solvant protique ajoutée au mélange initial est de 1 % à 300 % en poids par rapport au poids du mélange initial.

10. Le procédé selon l'une quelconque des revendications 1 à 9, dans lequel la distillation est effectuée à une pression de 0,05 bar à 1,2 bar.

11. Le procédé selon l'une quelconque des revendications 1 à 10, dans lequel la distillation est effectuée à une température de 80 °C à 150 °C.

12. Le procédé selon l'une quelconque des revendications 1 à 11, dans lequel le mélange de distillation est distillé pendant 0,5 heures à 48 heures.

13. Le procédé selon l'une quelconque des revendications 1 à 12, dans lequel un additif aprotique sélectionné parmi les hydrocarbures aliphatiques ayant 1 à 12 atomes de carbone, et leurs mélanges, est ajouté au mélange initial ou au mélange de distillation.

14. Le procédé selon la revendication 13, dans lequel l'additif aprotique est ajouté en une quantité pour arriver à une teneur en additif aprotique dans le mélange de distillation de 1 à 400 % en poids par rapport au poids du mélange de distillation.

15. Le procédé selon l'une quelconque des revendications 1 à 14, dans lequel la distillation est effectuée à l'aide d'une colonne de distillation.
